# EUROPÄISCHE PATENTSCHRIFT

(11) **EP 0 931 049 B1**
(45) Veröffentlichungstag und Bekanntmachung des Hinweises auf die Patenterteilung: **10.07.2002**
(21) Anmeldenummer: 97944793.5
(22) Anmeldetag: 26.08.1997
(51) Int. Cl.: C07C 57/04, C07C 51/44, B01D 1/30

(54) **VERFAHREN ZUR DESTILLATIVEN ABTRENNUNG VON REIN-(METH)ACRYLSÄURE AUS GEMISCHEN**
PROCESS FOR SEPARATING BY DISTILLATION PURE (METH)ACRYLIC ACID FROM MIXTURES
PROCEDE POUR LA SEPARATION PAR DISTILLATION D'ACIDE (METH)ACRYLIQUE PUR CONTENU DANS DES MELANGES

(30) Priorität: 27.08.1996 DE 19634614
(43) Veröffentlichungstag der Anmeldung: 28.07.1999
(73) Patentinhaber: BASF AKTIENGESELLSCHAFT, 67056 Ludwigshafen (DE)
(72) Erfinder: KROKER, Ruprecht, D-67240 Bobenheim-Roxheim (DE); WIEDEMANN, Manfred, D-67071 Ludwigshafen (DE)
(74) Vertreter: Isenbruck, Günter
(86) Internationale Anmeldenummer: EP9704639
(87) Internationale Veröffentlichungsnummer: WO9808798

(56) Entgegenhaltungen:
- DE-A- 2 027 357
- DE-A- 19 539 295
- GB-A- 2 021 426

## Beschreibung

Die vorliegende Erfindung betrifft ein Verfahren zur destillativen Abtrennung von Rein-(Meth)acrylsäure aus Gemischen, die (Meth)acrylsäure sowie Di- und Oligomere der (Meth)acrylsäure enthalten und im wesentlichen frei sind von Aldehyden und anderen Komponenten, deren Siedepunkt niedriger als der der (Meth)acrylsäure ist.

(Meth)acrylsäure wird als verkürzte Schreibweise verwendet und steht für Acrylsäure oder Methacrylsäure. (Meth)acrylsäure, entweder für sich oder in Form ihrer Ester, ist insbesondere zur Herstellung von Polymerisaten für die verschiedensten Anwendungsgebiete, z.B. Verwendung als Klebstoff, von Bedeutung und weist insbesondere im flüssigen Aggregatzustand eine hohe Polymerisationsneigung auf. Sichere Lagerung von im wesentlichen reiner flüssiger (Meth)acrylsäure ist selbst bei tiefen Temperaturen nur unter Zusatz von Polymerisationsinhibitor möglich.

Unter anderem ist (Meth)acrylsäure durch katalytische Gasphasenoxidation von Alkanen, Alkanolen, Alkenen oder Alkenalen erhältlich, die 3 bzw. 4 C-Atome enthalten. Besonders vorteilhaft ist (Meth)acrylsäure z.B. durch katalytische Gasphasenoxidation von Propen, Acrolein, tert.-Butanol, iso-Buten, iso-Butan, iso-Butyraldehyd oder Methacrolein erhältlich.

Als Ausgangsverbindungen sind aber auch solche denkbar, aus welchen sich die eigentliche C₃-/C₄-Ausgangsverbindung während der Gasphasenoxidation erst intermediär bildet. Beispielhaft genannt sei der Methylether des tert.-Butanols.

Dabei werden diese Ausgangsgase, in der Regel mit inerten Gasen wie Stickstoff, CO₂, gesättigten Kohlenwasserstoffen und/oder Wasserdampf verdünnt, im Gemisch mit Sauerstoff bei erhöhten Temperaturen (üblicherweise 200 bis 400°C) sowie ggf. erhöhtem Druck über übergangsmetallische (z.B. Mo, V, W und/oder Fe enthaltende) Mischoxidkatalysatoren geleitet und oxidativ in die (Meth)acrylsäure umgewandelt (vgl. z.B. DE-A 44 05 059, EP-A 253 409, EP-A 92 097, DE-A 44 31 949).

Aufgrund zahlreicher im Verlauf der katalytischen Gasphasenoxidation erfolgender Parallel- und Folgereaktionen sowie aufgrund der mitzuverwendenden inerten Verdünnungsgase wird bei der katalytischen Gasphasenoxidation jedoch keine Rein-(Meth)acrylsäure, sondern ein Reaktionsgemisch erhalten, das im wesentlichen (Meth)acrylsäure, die inerten Verdünnungsgase und Nebenprodukte enthält, aus welchem die (Meth)acrylsäure abgetrennt werden muß.

Üblicherweise erfolgt die Abtrennung der (Meth)acrylsäure aus dem Reaktionsgemisch über extraktive und destillative Trennverfahren. In der Regel wird dabei die gebildete (Meth)acrylsäure aus dem Reaktionsgemisch der Gasphasenoxidation zunächst in ein geeignetes Absorbtionsmittel aufgenommen. Durch destillative Auftrennung des Absorbats wird dann üblicherweise eine Roh-(Meth)acrylsäure erhalten, aus der mittels Durchlaufen weiterer destillativer Trennstufen häufig eine Rein-(Meth)acrylsäure erzeugt wird (vgl. z.B. DE-A 44 36 243, DE-C 21 36 396, DE-A 43 08 087, EP-A 297 445, EP-A 117 146, EP-B 102 642, GB-PS 1 346 737 und die DE-PS 2 207 184).

In all diesen rektifikativen Trennverfahren tritt, unabhängig davon, ob die (Meth)acrylsäureabtrennung über Kopf oder über Sumpf erfolgt, während der rektifikativen Abtrennung, auch bei Mitverwendung von Polymerisationsinhibitoren eine Belagsbildung innerhalb der Rektifikationsvorrichtungen nach relativ kurzer Zeit auf, weshalb die üblicherweise kontinuierlich durchgeführte Rektifikation von Zeit zu Zeit zum Zweck der Entfernung der Belagsbildung unterbrochen werden muß.

In jüngerer Zeit wurden umfangreiche Untersuchungen durchgeführt, um das Problem der Belagbildung bei der Destillation zur Abtrennung von (Meth)acrylsäure aus den bei der (Meth)acrylsäureherstellung anfallenden Reaktions- bzw. Produktgemischen zu verringern.

So beschreibt die DE 195 01 326.3 ein Verfahren der rektifikativen Abtrennung von (Meth)acrylsäure aus einem (Meth)acrylsäure als Hauptbestandteil und niedere Aldehyde als Nebenbestandteile enthaltenden Gemisch in einer aus Abtriebsteil und Verstärkerteil bestehenden Rektifikationskolonne, wobei man das die rektifikativ abzutrennende (Meth)acrylsäure umfassende Einsatzgemisch der Rektifikationskolonne nicht unmittelbar zuführt, sondern zunächst in ein brüdenseitig mit dem Verstärkerteil der Rektifikationskolonne verbundenes beheiztes Verweilzeitgefäß leitet, in dem das Einsatzgemisch am Sieden gehalten wird und anstelle des Einsatzgemisches als solchem der Rektifikationskolonne die Sumpfflüssigkeit des Verweilzeitgefäßes zuführt. Im Rahmen dieses Verfahrens werden in der Regel flüssige Gemische verarbeitet, die einen (Meth)acrylsäuregehalt von 5 bis 25 Gew.-% aufweisen, wie sie in der Regel nach der Versetzung des aus der katalytischen Gasphasenoxidation erhaltenen Reaktionsgemischs mit einem Absorptionsmittel und anschließender Desorption als Ablauf der Desorptionskolonne erhalten werden. Neben (Meth)acrylsäure enthalten diese Gemische noch große Mengen an Absorptionsmittel und niedere Aldehyde als Nebenkomponenten.

Ein jüngeres Verfahren zur kontinuierlichen destillativen Auftrennung von flüssigen Gemischen, die als Hauptbestandteil (Meth)acrylsäure enthalten, beschreibt die DE 195 39 295.7, wobei auch dort die Problematik der Verringerung der Belagsbildung während der Rektifikation im Vordergrund steht. Gemäß dieser Anmeldung wird die destillative Auftrennung eines Gemischs, das in der Regel einen Gehalt an (Meth)acrylsäure von ≥ 95 Gew.-% (Roh-(Meth)acrylsäure) aufweist in einer Destillationsvorrichtung, die eine Destillierblase, einen Kondensator und eine Verbindung zwischen Destillierblase und Kondensator aufweist und der das aufzutrennende flüssige Gemisch kontinuierlich zugeführt wird, durchgeführt, wobei im Rahmen dieses Verfahrens ein Anteil der Destillierblasenflüssigkeit überhitzt entnommen wird und unter Entspannung in die Destillationsvorrichtung zurückgeführt wird.

All den im Stand der Technik beschriebenen Verfahren ist gemeinsam, daß das bei den Destillationen bzw. Rektifikationen verbleibende Sumpfprodukt, das bis zu 99 Gew.-% (Meth)acrylsäure als Wertsubstanz enthält, nicht ebenfalls destillativ aufgearbeitet wird. Bislang wurde das erhaltene Sumpfprodukt, das einen relativ hohen Anteil an (Meth)acrylsäure in Form von Oligomeren enthält im Rahmen einer Koppelproduktion für die Butyl(meth)-acrylat-Herstellung verwendet. Eine derartige Koppelproduktion ist jedoch nicht immer möglich bzw. wirtschaftlich.

Demgemäß lag eine Aufgabe der vorliegenden Erfindung darin, ein Verfahren bereitzustellen, das in der Lage ist, in möglichst einfacher Weise und mit hoher Ausbeute die im Sumpfprodukt vorhandene (Meth)acrylsäure als Rein-(Meth)acrylsäure zu isolieren. Ein weiteres mit Hilfe dieses Verfahrens zu erreichendes Ziel bestand darin, neben möglichst viel Rein(Meth)acrylsäure gleichzeitig eine entsprechend geringe Menge an zu deponierendem bzw. zu verbrennendem Rückstand zu erhalten.

Im Rahmen der zu dieser Erfindung führenden Untersuchungen wurde überraschenderweise gefunden, daß die oben dargestellte Aufgabe durch ein einfaches Destillationsverfahren gelöst werden kann.

Demgemäß betrifft die vorliegende Erfindung ein Verfahren zur destillativen Abtrennung von Rein-(Meth)acrylsäure aus Gemischen, die (Meth)acrylsäure sowie Di- und Oligomere der (Meth)acrylsäure enthalten und im wesentlichen frei sind von Aldehyden und niedriger als (Meth)acrylsäure siedenden Komponenten, wie z.B. Wasser und Essigsäure, unter Verwendung einer Destillationsvorrichtung, die einen Dünnschichtverdampfer, einen Kondensator und eine eine Prallvorrichtung aufweisende Verbindung zwischen Dünnschichtverdampfer und Kondensator aufweist.

Der im Rahmen der vorliegenden Anmeldung verwendete Begriff "Roh(Meth)acrylsäure" bezeichnet eine (Meth)acrylsäure, die im allgemeinen eine Reinheit von ≥ 95 Gew.-%, häufig ≥ 97 Gew.-% aufweist, wobei sich die Verunreinigungen insbesondere aus niederen Aldehyden (z.B. Formaldehyd, Acetaldehyd, Acrolein, Methacrolein, Propionaldehyd, n-Butyr-aldehyd, Benzaldehyd, Furfural oder Crotonaldehyd), Wasser, niederen Alkancarbonsäuren (z.B. Essigsäure und Propionsäure) und Anhydriden von Alkancarbonsäuren (z.B. Maleinsäureanhydrid) rekrutieren.

Der im Rahmen der Anmeldung verwendete Begriff "Rein-(Meth)acrylsäure" bezeichnet eine (Meth)acrylsäure, deren Reinheit bei ≥ 98 Gew.-%, häufig bei ≥ 99 Gew.-% liegt.

Der im Rahmen der vorliegenden Anmeldung verwendete Begriff "Rest(Meth)acrylsäure" bezeichnet die im bei der destillativen Herstellung von Rein-(Meth)acrylsäure erhaltenen Sumpfprodukt verbliebene restliche (Meth)acrylsäure.

Das hier zu behandelnde Gemisch weist in der Regel einen Gehalt an Rest(Meth)acrylsäure von maximal 99 Gew.-% auf, wobei der Gehalt in der Regel zwischen 20 bis 99, vorzugsweise 70 bis 95 Gew.-% beträgt.

Der Gehalt an Di- und Oligomeren der (Meth)acrylsäure beträgt in der Regel maximal ungefähr 5000 ppm, vorzugsweise maximal ungefähr 1000 ppm, wobei der Gehalt an Di- und Oligomeren der (Meth)acrylsäure mit der Lagerdauer und/oder der Lagertemperatur ansteigt.

Als weitere Komponenten enthält das Gemisch Prozeßstabilisatoren, die die Polymerisation von (Meth)acrylsäure inhibieren, wie z.B. Luft, Hydrochinon, Hydrochinonmonomethylether, p-Nitrosophenol, p-Methoxyphenol oder Phenothiazin sowie Gemische aus zwei oder mehr davon, wobei Phenothiazin bevorzugt eingesetzt wird, in einer Menge von 50 bis 1000 ppm.

Normalerweise beträgt die Menge des Prozeßstabilisators (Polymerisationsinhibitors), bezogen auf die (Meth)acrylsäure (Gewicht) 200 bis 800 ppm.

Aufgrund der die Polymerisation von (Meth)acrylsäure inhibierenden Wirkung von Luftsauerstoff ist es vorteilhaft, die erfindungsgemäß verwendete Destillationsvorrichtung luftdurchströmt zu betreiben.

Ferner sind im Gemisch noch Aldehyd-Aminoguanidin-Adukte bzw. Aldehydhydrazin-Adukte in einer Menge von bis zu ungefähr 2000 ppm vorhanden, die vom zu Beginn der Aufarbeitung des bei der Gasphasenoxidation erhaltenen Reaktionsgemischs zugegebenen Aminoguanidinhydrocarbonat und/oder Hydrazin(derivaten) herrühren.

Der im Rahmen der vorliegenden Erfindung verwendete Ausdruck "im wesentlichen frei von" bedeutet, daß der Gehalt der Aldehyde sowie der "weiteren Komponenten", auf die in diesem Zusammenhang Bezug genommen wird, insbesondere der Aldehyde und/oder Niedrigsieder, wie z.B. Wasser und niedere Alkancarbonsäuren, bei jeweils maximal 1000 ppm, vorzugsweise ungefähr 500 ppm und insbesondere ungefähr 300 ppm liegt.

Besonders vorteilhaft wird das erfindungsgemäße Verfahren bei der Aufarbeitung eines bei der destillativen Herstellung von Rein-(Meth)acrylsäure erhaltenenen Sumpfprodukts mit Rest-(Meth)acrylsäure eingesetzt.

Im Rahmen des erfindungsgemäßen Verfahrens ist es wesentlich, daß die Destillationsvorrichtung als Dünnschichtverdampfer (Sambay- oder Luva-Verdampfer) ausgelegt ist (vgl. z.B. GB-A-2 021 426 und DE-A-2 027 357), wobei dieser selbstverständlich ein- oder mehrstufig ausgeführt sein kann. Die Ausführung als Dünnschichtverdampfer ist für die vorteilhafte Durchführung des Verfahrens insoweit wesentlich, als daß mit anderen Verdampfern, wie z.B. Röhrenverdampfern, die hier aufzuarbeitende meist relativ viskosen Gemische, die im allgemeinen eine Viskosität von ungefähr 50 bis ungefähr 1000 mPas bei 50°C, nur schwer aufzuarbeiten sind und bereits nach relativ kurzer Zeit Belagsbildung bzw. Verkrustung der Apparatur zu beobachten ist. Als im Rahmen der vorliegenden Erfindung verwendbare Dünnschichtverdampfer sind neben den oben erwähnten auch Fallfilm-Verdampfer und Filmtruder zu nennen.

Weiterhin weist die erfindungsgemäß zu verwendende Vorrichtung eine eine Prallvorrichtung aufweisende Verbindung zwischen dem Dünnschichtverdampfer und dem Kondensator auf. Abgesehen von der Prallvorrichtung, die das Mitreißen von Flüssigkeitströpfchen, insbesondere vom Polymerisationsinhibitor, verhindert, ist die Verbindung im wesentlichen frei von weiteren Einbauten. Als Prallvorrichtung sind Demistoren, d.h. Drahtgestrick-Packungen mit sehr großer innerer Oberfläche, die z.B. aus Chromnickelstählen, Aluminium, Kupfer, Nickel, Polypropylen, Polytetrafluorethylen usw. gefertigt sein können, sowie eine einfache Packung aus Raschigringen usw. verwendbar.

Um die Temperaturen während der Aufarbeitung möglichst gering zu halten, erfolgt die erfindungsgemäße destillative Abtrennung der (Meth)acrylsäure vorzugsweise bei reduziertem Druck. Dabei wird erfindungsgemäß zweckmäßigerweise bei einem Druck ≤ 5 x 10⁴ Pa, vatzugsweise bei 0,1 bis 5 x 10⁴ Pa, weiter bevorzugt bei 0,1 bis 3 x 10⁴ Pa und insbesondere bei ungefähr 7 x 10³ Pa gearbeitet. Die Temperaturen im Dünnschichtverdampfer betragen in der Regel 40 bis 140°C, vorzugsweise 40 bis 100°C und insbesondere ungefähr 70°C, während die entsprechende Wandtemperatur des Dünnschichtverdampfers um 30 bis 40°C höher liegt.

Vorzugsweise wird das oben beschriebene erfindungsgemäße Verfahren zur destillativen Abtrennung von Rein-(Meth)acrylsäure mit den üblichen Verfahren zur Gewinnung von Rein-(Meth)acrylsäure aus Reaktionsgemischen, die bei der Herstellung von (Meth)acrylsäure erhalten werden, wie eingangs bei der Diskussion des Standes der Technik erwähnt, gekoppelt.

Demgemäß betrifft die vorliegende Erfindung weiterhin ein Verfahren zur destillativen Gewinnung von Rein-(Meth)acrylsäure, das die folgenden Stufen (a) und (b) umfaßt:
(a) Abtrennung über extraktive und destillative Trennverfahren von Rein(Meth)acrylsäure aus Reaktionsgemischen, die bei der Herstellung von (Meth)acrylsäure erhalten werden, und
(b) Behandeln eines in Schritt (a) in einem destillativen Trennverfahren erhaltenen Sumpfprodukts enthaltend (Meth)acrylsäure mit einem Verfahren zur destillativen Abtrennung von Rein-(Meth)acrylsäure aus Gemischen, wie oben definiert.

Sowohl in Schritt (a) als auch in Schritt (b) wird Rein-(Meth)acrylsäure jeweils über Kopf, oder seitlich (Schritt (a)) und über Kopf (Schritt (b)) erhalten.

Dabei können die in Schritt (a) und (b) erhaltenen Produktströme aus Rein(Meth)acrylsäure vereinigt werden.

Im allgemeinen wird die als Produkt erhaltene Rein-(Meth)acrylsäure mit einem Lagerstabilisator, wie z.B. Hydrochinonmonomethylether, versetzt.

In einer besonders bevorzugten Ausführungsform der vorliegenden Erfindung wird Schritt (a) wie folgt durchgeführt:

Zunächst wird eine Roh-(Meth)acrylsäure nach einem Verfahren gemäß des Standes der Technik erhalten, wie z.B. in der EP 95 118 901 (EP-A 0 717 029) beschrieben.

Die destillative Herstellung von Rein-(Meth)acrylsäure gemäß Schritt (a) erfolgt dann durch eine einstufige Destillation, bei der Rein-(Meth)acrylsäure über Kopf abgenommen wird, in einer Destillationsvorrichtung, die eine Destilliereinrichtung, einen Kondensator und eine Verbindung zwischen Destilliereinrichtung und Kondensator aufweist und der das aufzutrennende flüssige, Roh-(Meth)acrylsäure enthaltende Gemisch kontinuierlich zugeführt wird, wobei wenigstens ein Teil der zum Siedeverdampfen des flüssigen Gemisches erforderlichen Energie der Destillationsvorrichtung dadurch zugeführt wird, daß man dem bei einem Druck Pₓ befindlichen flüssigen Inhalt der Destilliereinrichtung kontinuierlich einen Anteil entnimmt, diesen bei einem oberhalb von Pₓ gelegenen Druck P_{y} auf eine Temperatur T_{y}, mit der Maßgabe erhitzt, daß T_{y}, oberhalb der zum Druck Pₓ gehörigen Siedetemperatur Tₓ und unterhalb der zum Druck P_{y} gehörigen Siedetemperatur T_{y} des flüssigen Inhalts der Destilliereinrichtung liegt, und anschließend den bezogen auf den Druck Pₓ so überhitzten und entnommenen Anteil der Flüssigkeit der Destilliereinrichtung in die Destillationsvorrichtung zurückführt. Ein derartiges Verfahren ist in der DE 195 39 295.7 beschrieben.

Das bei diesem Verfahren als Sumpfprodukt zurückbleibende Gemisch kann dann direkt in eine zweite Vorrichtung, z.B. über Pumpen, geführt werden und entsprechend, wie vorstehend beschrieben, als Schritt (b) aufgearbeitet werden.

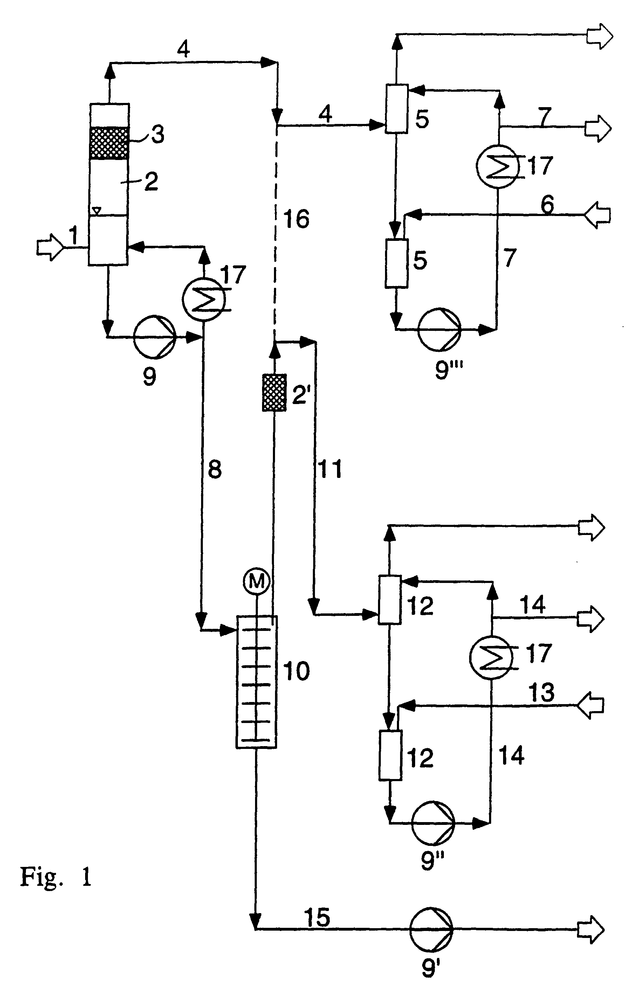

Eine schematische Darstellung dieses Verfahrens ist in Fig. 1 gezeigt.

Dabei wird die aus der Extraktion gewonnene Roh-(Meth)acrylsäure, die mit Aminoguanidinhydrogencarbonat (AGHC) und/oder Hydrazin zum Abbau der Aldehyde behandelt wurde und Polymerisationsinhibitoren und ein Netzmittel, wie z.B. Dodecylbenzolsulfonsäure als Antifouling-Mittel enthält, über Leitung (1) in eine Destillationsapparatur (2), die ggf. mit einem Demistor (3) versehen ist, eingebracht und destilliert, wobei die Rein-(Meth)acrylsäure über Kopf abgenommen wird und mittels der Leitung (4) in einem Kühlkreislauf (5) kondensiert, über die Zuleitung (6) mit einem Stabilisator versehen wird. Über die Leitung (7), die (wie dargestellt) mit einer Umwälzpumpe (9"') versehen sein kann, wird das so erhaltene, stabilisierte Reinprodukt in einen nicht dargestellten Behälter überführt.

Das bei der Destillation in der Vorrichtung (2) erhaltene Sumpfprodukt mit Rest-(Meth)acrylsäure wird abgezogen und über eine Zuleitung (8), die ggf. mit einer Umwälzpumpe (9) versehen sein kann, in den Dünnschichtverdampfer (10) überführt, der mit einem Demistor (2') versehen ist, und wird dort einer Rückstandsdestillation unterzogen. Rein-(Meth)acrylsäure wird wiederum über Kopf abgenommen und über die Zuleitung (11) einem weiteren Kühlkreislauf (12) überführt und dort ebenfalls mit einem Stabilisator über die Zuführleitung (13) versetzt. Das so erhaltene Reinprodukt wird über die Leitung (14), die (wie dargestellt) mit einer Umwälzpumpe (9") versehen sein kann, in einen hier nicht dargestellten Behälter überführt. Der sich im Dünnschichtverdampfer befindliche Rückstand, der hochsiedende Verbindungen enthält, wird über die Abführleitung (15), die eine Austragspumpe (9') enthält, kontinuierlich aus dem Dünnschichtverdampfer entfernt und der Verbrennung zugeführt. Die anfallende Rückstandsmenge beträgt, bezogen auf den Einsatz von Roh-(Meth)acrylsäure, ca. 1 - 2 Gew.-%.

Wie in Fig. 1 ebenfalls schematisch durch die gestrichelte Linie (16) dargestellt, kann die bei der Rückstandsdestillation am Kopf abgenommene Rein(Meth)acrylsäure auch über eine Verbindung mit Kühlvorrichtung (16) mit dem ebenfalls aus Rein-(Meth)acrylsäure bestehenden Produktstrom der Destillation der Vorrichtung (2) vereinigt werden und beide Produktströme gemeinsam kondensiert, mit einem Lagerstabilisator versehen und in einen hier nicht dargestellten Behälter überführt werden.

Ferner können - wie ebenfalls dargestellt - an verschiedenen Stellen des Verfahrens Umlaufverdampfer (17) vorhanden sein.

Dabei ist insgesamt zu beachten, daß das erfindungsgemäße Verfahren bevorzugt kontinuierlich aber auch diskontinuierlich durchgeführt werden kann und zwar entweder für sich alleine oder gekoppelt mit anderen Verfahrensstufen innerhalb eines Verfahrens zur Herstellung von Rein-(Meth)acrylsäure durch Gasphasenoxidation und anschließender Aufreinigung.

### Beispiel

Ein bei der Herstellung durch katalytische Gasphasenoxidation von Acrolein und anschließender extraktiver und destillativer Aufreinigung derselben erhaltenes Sumpfprodukt, das die folgende Zusammensetzung aufwies:

| | |
|---|---|
| 92 Gew.-% | Acrylsäure |
| 4.000 ppm | Phenothiazin (Prozeß-Polymerisationsinhibitor) |
| 2 Gew.-% | Di- und Oligomere der Acrylsäure |
| 2 Gew.-% | Additionsprodukte von AGHC und Aldehyden/Maleinsäureanhydrid |
| 3 Gew.-% | Dodecylbenzolsulfonsäure (Antifouling-Mittel) |
| 300 ppm | Propionsäure |
| 300 ppm | Essigsäure |
| 100 ppm | Wasser |

wurde einer Destillationsvorrichtung mit Dünnschichtverdampfer und einer Prallvorrichtung zugeführt.

Die genauen Daten der Destillationsvorrichtung und der Betriebsweise lauteten:
- Dünnschichtverdampfer vom Sambay-Typ mit einer Wandfläche von 10 m²
- Demistor mit 700 mm Durchmesser und einer Länge von 1 m
- Zuführung des aufzutrennenden Gemischs unmittelbar in den Dünnschichtverdampfer mit einer Zulauftemperatur von 30 bis 90 °C in einer Menge von 0,5 m³/h;
- Druck in der Vorrichtung: 7 x 10³ Pa (70 mbar);
- Siedetemperatur am Kopf der Vorrichtung: ungefähr 70°C;
- Kühlkreis 80 m³/h.

Im Kondensator fiel ein Kondensat an, das 99,7 Gew.-% Acrylsäure sowie < 1.000 ppm Diacrylsäure, ca. 300 ppm Propionsäure, ca. 300 ppm Essigsäure, < 10 ppm niedermolekulare Aldehyde und < 1 ppm Phenothiazin enthielt und dem unmittelbar 200 ppm Hydrochinonmonomethylether als Lagerstabilisator zugesetzt wurde.

Die Ausbeute an Acrylsäure, bezogen auf die eingesetzte Menge, lag bei ungefähr 90 %. Das bei dieser Destillation in einer Menge von ungefähr 10 %, bezogen auf die eingesetzte Gesamtmenge, anfallende Sumpfprodukt bestand im wesentlichen aus Di- und Olygomeren der Acrylsäure, Prozeßstabilisator, Antifouling-Mittel, den Additionsprodukten von AGHC und Aldehyden/Maleinsäureanhydrid, sowie einer geringen Menge an Acrylsäure.

## Patentansprüche

1. Verfahren zur destillativen Abtrennung von Rein-(Meth)acrylsäure aus Gemischen, die (Meth)acrylsäure sowie Di- und Oligomere der (Meth)acrylsäure enthalten und im wesentlichen frei sind von Aldehyden und niedriger als (Meth)acrylsäure siedenden Komponenten
unter Verwendung einer Destillationsvorrichtung, die
einen Dünnschichtverdampfer,
einen Kondensator und
eine eine Prallvorrichtung aufweisende Verbindung zwischen Dünnschichtverdampfer und Kondensator aufweist.

2. Verfahren nach Anspruch 1, wobei das Gemisch ein bei der destillativen Herstellung von Rein-(Meth)acrylsäure erhaltenes Sumpfprodukt mit Rest-(Meth)acrylsäure ist.

3. Verfahren nach Anspruch 1 oder 2, wobei der Anteil der Rest(Meth)acrylsäure im Gemisch bis zu 99 Gew.-% beträgt.

4. Verfahren nach einem der vorhergehenden Ansprüche, wobei das Gemisch als Polymerisationsinhibitor Phenothiazin, Hydrochinon, Hydrochinonmonomethylether, p-Nitrosophenol, p-Methoxyphenol oder Gemische aus zwei oder mehr davon enthält.

5. Verfahren nach einem der vorhergehenden Ansprüche, wobei der Druck im Dünnschichtverdampfer 1 x 10³ bis 3 x 10⁴ Pa beträgt.

6. Verfahren nach einem der vorhergehenden Ansprüche, wobei die Temperatur am Kopf des Dünnschichtverdampfers während der Destillation 40 bis 140°C beträgt.

7. Verfahren zur destillativen Gewinnung von Rein-(Meth)acrylsäure, umfassend die folgenden Stufen (a) und (b):
(a) Abtrennung über extraktive und destillative Trennverfahren von Rein-(Meth)acrylsäure aus Reaktionsgemischen, die bei der Herstellung von (Meth)acrylsäure erhalten werden, und
(b) Behandeln eines in Schritt (a) bei einem destillativen Trennverfahren erhaltenen Sumpfprodukts enthaltend Rest-(Meth)acrylsäure mit einem Verfahren nach einem der Ansprüche 2 bis 6.

8. Verfahren nach Anspruch 7, wobei die Abtrennung von Rein(Meth)acrylsäure gemäß Schritt (a) in einer Destillationsvorrichtung, die eine Destilliereinrichtung, einen Kondensator und eine Verbindung zwischen Destilliereinrichtung und Kondensator aufweist und der das aufzutrennende flüssige Gemisch kontinuierlich zugeführt wird, durchgeführt wird, wobei mindestens ein Teil der zum Siedeverdampfen des flüssigen Gemisches erforderlichen Energie der Destillationsvorrichtung dadurch zugeführt wird, daß man dem bei einem Druck Pₓ befindlichen flüssigen Inhalt der Destilliereinrichtung kontinuierlich einen Anteil entnimmt, diesen bei einem oberhalb von Pₓ gelegenen Druck P_{y} auf eine Temperatur T_{y}, mit der Maßgabe erhitzt, daß T_{y}, oberhalb der zum Druck Pₓ gehörigen Siedetemperatur Tₓ und unterhalb der zum Druck P_{y} gehörigen Siedetemperatur T_{y} des flüssigen Inhalts der Destilliereinrichtung liegt, und anschließend den, bezogen auf den Druck Pₓ überhitzten, entnommenen Anteil der Flüssigkeit der Destilliereinrichtung in die Destillationsvorrichtung zurückführt.

9. Verfahren nach Anspruch 7 oder 8, wobei die in Schritt (a) und (b) erhaltenen Produktströme aus Rein-(Meth)acrylsäure vereinigt werden.

10. Verfahren nach einem der vorhergehenden Ansprüche, wobei die als Produkt erhaltene Rein-(Meth)acrylsäure mit einem Lagerstabilisator versetzt wird.

## Claims

1. A process for distillative separation of pure (meth)acrylic acid from mixtures which comprise (meth)acrylic acid and dimers and oligomers of (meth)acrylic acid and are essentially free from aldehydes and from components whose boiling point is lower than that of (meth)acrylic acid, using a distillation apparatus which has a thin-film evaporator, a condenser and a connection which contains a baffle device and links the thin-film evaporator and the condenser.

2. A process as claimed in claim 1, where the mixture is a bottom product obtained in the distillative preparation of pure (meth)acrylic acid, containing residual (meth)acrylic acid.

3. A process as claimed in claim 1 or 2, where the proportion of residual (meth)acrylic acid in the mixture is up to 99% by weight.

4. A process as claimed in any of the preceding claims, where the mixture comprises, as polymerization inhibitor, phenothiazine, hydroquinone, hydroquinone monomethyl ether, p-nitrosophenol, p-methoxyphenol or mixtures of two or more thereof.

5. A process as claimed in any of the preceding claims, where the pressure in the thin-film evaporator is from 1 x 10³ to 3 x 10⁴ Pa.

6. A process as claimed in any of the preceding claims, where the temperature at the top of the thin-film evaporator during distillation is from 40 to 140°C.

7. A process for distillative recovery of pure (meth)-acrylic acid, comprising the following stages (a) and (b):
(a) separation by way of extractive and distillative separation techniques of pure (meth)acrylic acid from reaction mixtures obtained in connection with the preparation of (meth)-acrylic acid, and
(b)treatment of a bottom product, obtained in step (a) in a distillative separation technique and comprising (meth)acrylic acid, by a process as claimed in any of claims 2 to 6.

8. A process as claimed in claim 7, where the separation of pure (meth)acrylic acid in accordance with step (a) is conducted in a distillation apparatus which has a distillation device, a condenser and a connection between the distillation device and the condenser and to which the liquid mixture that is to be separated is supplied continuously, where at least part of the energy required to evaporate the liquid mixture is supplied to the distillation apparatus by continuously withdrawing from the distillation device a fraction of its liquid contents which are at a pressure Pₓ, heating this fraction to a temperature T_{y}, and a pressure P_{y} which is above Pₓ, with the proviso that T_{y'} is above the boiling temperature Tₓ of the liquid contents of the distillation device when subject to the pressure Pₓ and is below the boiling temperature T_{y} of the liquid contents of the distillation device when subject to the pressure P_{y}, and passing the liquid fraction withdrawn from the distillation device and superheated in this manner, relative to the pressure Pₓ, back to the distillation apparatus.

9. A process as claimed in claim 7 or 8, where the product streams of pure (meth)acrylic acid obtained in step (a) and (b) are combined.

10. A process as claimed in any of the preceding claims, where a storage stabilizer is added to the pure (meth)acrylic acid obtained as product.

## Revendications

1. Procédé de séparation par distillation de l'acide (méth)acrylique pur, à partir des mélanges contenant l'acide (méth)acrylique ainsi que les dimères et les oligomères de l'acide (méth)acrylique et qui sont, pour l'essentiel, exempt d'aldéhydes et de composants qui présentent un point d'ébullition inférieur à celui de l'acide (méth)acrylique, en employant un dispositif de distillation comprenant
un évaporateur à couche mince,
un condenseur et
un raccordement qui comprend un dispositif à chicanes et qui est situé entre l'évaporateur à couche mince et le condenseur.

2. Procédé selon la revendication 1, **caractérisé en ce que** le mélange est constitué d'un produit de fond obtenu au moyen de la préparation par distillation de l'acide (méth)acrylique et contenant l'acide (méth)acrylique résiduel.

3. Procédé selon la revendication 1 ou 2, **caractérisé en ce que** l'acide (méth)acrylique résiduel dans le mélange est représenté dans une proportion allant jusqu'à 99% en poids.

4. Procédé selon l'une des revendications précédentes, **caractérisé en ce que** le mélange contient, en tant qu'inhibiteur de polymérisation, le phénothiazine, l'hydroquinone, l'éther monométhylique d'hydroquinone, le p-nitrosophénol, le p-méthoxyphénol ou les mélanges constitués de deux ou de plusieurs de ces composés.

5. Procédé selon l'une des revendications précédentes, **caractérisé en ce que** la pression dans l'évaporateur à couche mince est comprise entre 1 x 10³ Pa et 3 x 10⁴ Pa.

6. Procédé selon l'une quelconque des revendications précédentes, **caractérisé en ce que** la température de tête de l'évaporateur à couche mince est comprise, pendant la distillation, entre 40°C et 140°C.

7. Procédé de préparation par distillation de l'acide (méth)acrylique pur, comprenant les étapes (a) et (b) suivantes :
(a) séparation de l'acide (méth)acrylique pur à partir de mélanges réactionnels que l'on obtient lors de la préparation de l'acide (méth)acrylique, au moyen de procédés de séparation par extraction et par distillation, et
(b) traitement, par un procédé selon l'une des revendication 2 à 6, du produit de fond contenant l'acide (méth)acrylique résiduaire et que l'on a obtenu dans l'étape (a) lors de l'emploi d'un procédé de séparation par distillation.

8. Procédé selon la revendication 7, **caractérisé en ce que** l'on procède à la séparation de l'acide (méth)acrylique pur selon l'étape (a), en ajoutant en continu le mélange liquide destiné à la séparation, dans un dispositif de distillation qui comprend une installation de distillation, un condenseur et un raccordement situé entre l'installation de distillation et le condenseur, en amenant, au dispositif de distillation, au moins une partie de l'énergie nécessaire pour effectuer l'évaporation par ébullition du mélange liquide, au moyen d'un prélèvement en continu d'une partie du contenu liquide du dispositif de distillation, contenu qui est maintenu à une pression Pₓ, partie que l'on chauffe, à une pression P_{y} qui est supérieure à Pₓ, pour parvenir à une température T_{y}, à la condition que T_{y} soit supérieure à la température d'ébullition Tₓ correspondant à la pression Pₓ, et inférieure à la température d'ébullition T_{y} du contenu liquide de l'installation de distillation correspondant à la pression P_{y}, et **en ce que** l'on recycle ensuite, dans le dispositif de distillation, la partie du liquide prélevée de l'installation de distillation et qui est surchauffée par rapport à la pression Pₓ.

9. Procédé selon la revendication 7 ou 8, **caractérisé en ce que** l'on réunit les courants de produits obtenus dans les étapes (a) et (b) constitués d'acide (méth)acrylique pur.

10. Procédé selon l'une quelconque des revendications précédentes, **caractérisé en ce que** l'on mélange un stabilisant de stockage à l'acide (méth)acrylique obtenu en tant que produit.
